# EUROPÄISCHE PATENTSCHRIFT

(11) **EP 1 877 028 B1**
(45) Veröffentlichungstag und Bekanntmachung des Hinweises auf die Patenterteilung: **08.04.2009**
(21) Anmeldenummer: 07702965.0
(22) Anmeldetag: 23.01.2007
(51) Int. Cl.: A61K 8/02, A61K 8/92, A61Q 19/10

(54) **CREMEREINIGUNGSTUCH**
CREAM CLEANING CLOTH
LINGETTE NETTOYANTE IMPRÉGNÉE DE CRÈME

(30) Priorität: 18.05.2006 DE 102006023254
(43) Veröffentlichungstag der Anmeldung: 16.01.2008
(73) Patentinhaber: FHW Feucht-Hygiene-Werk GmbH, 48607 Ochtrup (DE)
(72) Erfinder: TENBUSCH, Wolfgang, 28355 Bremen (DE); PULINA, Michael, 47807 Krefeld (DE); SCHUMACHER, Jörg, 48565 Steinfurt (DE)
(74) Vertreter: Ruschke, Hans Edvard
(86) Internationale Anmeldenummer: PCT/EP2007/000556
(87) Internationale Veröffentlichungsnummer: WO 2007/134649

(56) Entgegenhaltungen:
- WO-A-00/64409
- WO-A-03/005981
- WO-A-03/092635
- FR-A- 2 862 200

## Beschreibung

### Gebiet der Erfindung

Die vorliegende Erfindung betrifft ein Cremereinigungstuch mit einer nicht flüssigen, sondern pastösen bis einschließlich festen Lotion und einem Trägermaterial sowie ein Verfahren zu seiner Herstellung.

### Hintergrund der Erfindung und Stand der Technik

Es liegt in den Bedürfnissen der Menschheit, die Haut zu reinigen und zu pflegen. Insbesondere seit Mitte des 20. Jahrhunderts wurden neben den klassischen Seifen ("Rinse off") auch viele weitere Produkte zur Körperpflege und Körperreinigung entwickelt. Neben der festen Seife entstand die Flüssigseife, um nur ein Beispiel zu nennen.

Neben der eindeutigen Reinigung der Haut hat die Pflege einen immer bedeutsameren Stellenwert eingenommen. Daraus entwickelte sich eine hohe Nachfrage für kosmetische Erzeugnisse ("Leave on"), welche einen neuen Produktbereich bilden konnten. Dazu wird bevorzugt eine Pflegecreme mit speziellen Produktausrichtungen angeboten. Da diese Produkte nicht abgewaschen werden, ist es erforderlich, besonders milde und für die Haut nicht reizende Edukte einzusetzen.

In den letzten 20 Jahren wurden diese Produktideen erstmals auf feuchte Tücher übertragen. Neben dem feuchten Toilettenpapier wurden Produkte wie feuchte Babytücher und in jüngster Zeit auch feuchte Kosmetiktücher entwickelt.

Dabei ist zunächst zwischen dem Trägermaterial und den Lotionen zu unterscheiden, wobei die Lotionen wiederum in tensidische Systeme und Emulsionen zu unterteilen sind. Die tensidischen Systeme setzen sich zusammen aus Komponenten wie Parfümöl, Wasser, Konservierungsmitteln und Wirkstoffen, während die Emulsionen neben Parfümöl, Wasser, Konservierungsmitteln und Wirkstoffen auch Komponenten wie Öl und Emulgatoren enthalten. Die Emulsionen werden in Babytüchem und Kosmetiktüchern eingesetzt, da es auf Grund der reinigenden und pflegenden Eigenschaften unbedingt erforderlich ist, eine unpolare Phase einzuarbeiten.

Dies wurde bereits in den Produkten der Pflegecremes erkannt. Jedoch ist es bei einer Pflegecreme möglich, die unpolare Phase zu einem höheren Anteil einzuarbeiten, da sowohl die Emulgatoren als auch der Anteil der Ölphase erhöht werden können. Für ein feuchtes Pflegetuch (im primären Sinne der Hautreinigung) war dies derzeit noch nicht möglich, da die Technologie die Verarbeitung von hochviskosen Medien bisher nicht zuliess und die Tränkung eines Tuchs mit einer Creme nicht homogen erfolgen konnte.

Die Emulsionen wurden für den Einsatz in feuchten Tüchern daher bisher mit einer Viskosität im "wasserähnlichen" Milieu (< 2000 mPas) entwickelt und produziert.

Die DE 601 14 206 T2 offenbart zwar ein Produkt, welches aus einem Vlies oder Vergleichbarem in Kombination z.B. mit einer Creme besteht. Jedoch handelt es sich hier um ein sogenanntes Mehrphasenprodukt, d.h. nur ein Teil des Tuchvolumens ist mit Creme versehen, während ein anderer Teil trocken, d.h. nicht mit Creme getränkt, ist (sogenannte "Sandwichstruktur"). Ein Mehrphasenprodukt ist durch das Merkmal charakterisiert, dass es sich nicht um ein homogenes Produkt handelt, d.h. das Trägermaterial ist nicht vollständig mit Creme getränkt.

In der DE 200 01 165 U1 wird zwar der Begriff eines Cremetuchs verwendet, jedoch geht diese Druckschrift lediglich auf eine mögliche Verpackungslösung ein. Das Problem der Herstellung eines homogenen Cremetuchs im Sinne der vorliegenden Erfindung ist nicht offenbart.

In WO 00/64 409 A1 werden Gesichtstücher beschrieben, die mit Cremezusammensetzungen oberflächig impregniert werden.

Trägermaterialien im Bereich der Pflege sind die so genannten Spunlace-Gewebesorten. Dieser Begriff definiert die Herstellung des Trägermaterials. Verschiedene Substanzen (Baumwolle, Kunststofffasern, Viskose, u.a.), mindestens zwei, werden miteinander unter Wasserstrahl vermengt. Die Grammatur kann dabei von 30 - 120 g/m² variiert werden. Ob es sich dabei um eine glatte oder geprägte Oberflächenstruktur handelt, ist hier nicht von Bedeutung. Bestreben ist es lediglich, ein möglichst weiches Material zur sanften Pflege einzusetzen. Auf diesem Gebiet sind in den letzten Jahren entscheidende Fortschritte zu verzeichnen. So sind in letzter Zeit Spunlace-Materialien mit einem Baumwollanteil entwickelt worden, welche erstaunliche Ergebnisse bezüglich der Weichheit aufweisen. Die Weichheit bzgl. Rauhigkeit eines Trägermaterials entsteht in den Kernpunkten aus Beweglichkeit der Faser an der Oberfläche, Unebenheit der Oberfläche, sowie der Faserdicke und Faserdichte.

Im technisch relevanten Schritt wurden dann bisher die bekannten Emulsionen und das Trägermaterial miteinander kombiniert. Der Tränkungsgrad konnte dabei, je nach Grammatur, zwischen 100 und maximal 400 Gew.-% Lotion/Tuch variieren.

Da bisher also nur relativ niedrig-viskose Emulsionen befriedigend in ein Trägermaterial eingearbeitet werden konnten, besteht nach wie vor Bedarf an einem homogen mit einer Creme versehenen Tuch, mit dem eine intensivere Pflege erzielt werden kann.

### Zusammenfassung der Erfindung

Es ist das Ziel der vorliegenden Erfindung, eine Technologie vorzusehen, welche eine homogene Aufbringung einer Creme auf einem Tuch ermöglicht - ein damit zusammenhängender Aspekt besteht darin, die Weichheit zu erhöhen, indem die spürbare Oberflächenrauhigkeit durch Ausfüllen auch der Leerräume der Matrixstruktur durch die Creme gemindert wird. Darüber hinaus sollte das Cremetuch hautfreundlich sein, wodurch der Vorteil gegenüber den bisherigen Pflegetüchern (Kosmetiktücher, Babytücher) noch offenkundiger wird.

Dieses Ziel ist erreicht worden durch ein Verfahren nach Anspruch 1 bzw. ein Cremetuch nach Anspruch 5. Das Verfahren betrifft die Herstellung eines Cremetuchs, das ein Trägermaterial und ein hochviskoses Tränkungsmedium in Form einer Öl-/Wasser-Emulsion mit flüssigkristalliner Phase enthält, und ist durch die folgenden Schritte gekennzeichnet:
- Erwärmen des hochviskosen Tränkungsmediums, bis es flüssig und die flüssigkristalline Phase aufgeschmolzen ist;
- Aufbringen des nunmehr flüssigen Tränkungsmediums auf das Trägermaterial;
- Abkühlen des mit dem Tränkungsmedium versehenen Trägermaterials, bis das Tränkungsmedium wieder hochviskos ist und wieder eine flüssigkristalline Phase umfaßt, sodaß das Tränkungsmedium homogen über das Trägermaterial verteilt ist, indem das Tränkungsmedium vom Trägermaterial bei geringer Sättigung nur durch Adsorption in den Fasern, bei höherer Sättigung zusätzlich durch Bildung von flüssigkristallinen Phasen um die Fasern und bei vollständiger Sättigung zusätzlich durch Auffüllen der Zwischenräume im Trägermaterial aufgenommen wird.

Das Cremetuch umfasst demgemäss ein Trägermaterial und ein hochviskoses Tränkungsmedium in Form einer Öl-/Wasser-Emulsion mit flüssigkristalliner Phase und ist dadurch gekennzeichnet, dass das Tränkungsmedium homogen über das Trägermaterial verteilt ist, indem das Tränkungsmedium vom Trägermaterial bei geringer Sättigung nur durch Adsorption in den Fasern, bei höherer Sättigung zusätzlich durch Bildung von flüssigkristallinen Phasen um die Fasern und bei vollständiger Sättigung zusätzlich durch Auffüllen der Zwischenräume im Trägermaterial verteilt ist.

Vorteilhafte Ausgestaltungen des Verfahrens bzw. des Cremetuchs ergeben sich aus den Unteransprüchen 2-4 bzw. 6-8.

### Beschreibung der Figuren

- Fig. 1: zeigt das Phasenverhalten der Emulsionen in einer Temperatur-Viskositäts-Kurve;
- Fig. 2: zeigt ein Verfahrensfließbild des erfindungsgemäßen Herstellungsverfahrens;
- Fig. 3: zeigt das Hystereseverhalten bei der Ausbildung der flüssigkristallinen Phase;
- Fig. 4a: zeigt in einem Modellbild die Tränkung eines Feuchtuchs im Vergleich zu einem Cremetuch;
- Fig. 4b: zeigt die verschiedenen Sättigungsgrade bei der Tränkung eines Cremetuchs;
- Fig. 5a: zeigt eine schematische Darstellung der Oberfläche eines trockenen Trägermaterials; und
- Fig. 5b: zeigt eine schematische Darstellung der Oberfläche eines gequollenen Trägermaterials.

### Beschreibung der bevorzugten Ausführungsformen

Die Erfindung konnte dadurch realisiert werden, dass gezielt für den Einsatz in feuchten Tüchern Emulsionen (mit einer Viskosität > 2000mPas) entwickelt wurden, welche sich mit genauer Einstellung der Tränkung auf feuchte Tücher applizieren lassen. Beispielhaft wurde die in Tab. 1 gezeigte Formulierung eingesetzt.

Anhand dieses Beispiels wurden Emulsionen mit flüssigkristallinen Phasen studiert und charakterisiert.

Entscheidend für die Verwendbarkeit und damit für den durch die vorliegende Erfindung erreichten Fortschritt sind die folgenden physikalischen Parameter: Schmelztemperatur T_{S}, Erstarrungstemperatur T_{E}, Zersetzungstemperatur T_{Z} und Schmelzbereich dT_{S}, sowie das Viskositätsverhalten der Emulsionen.

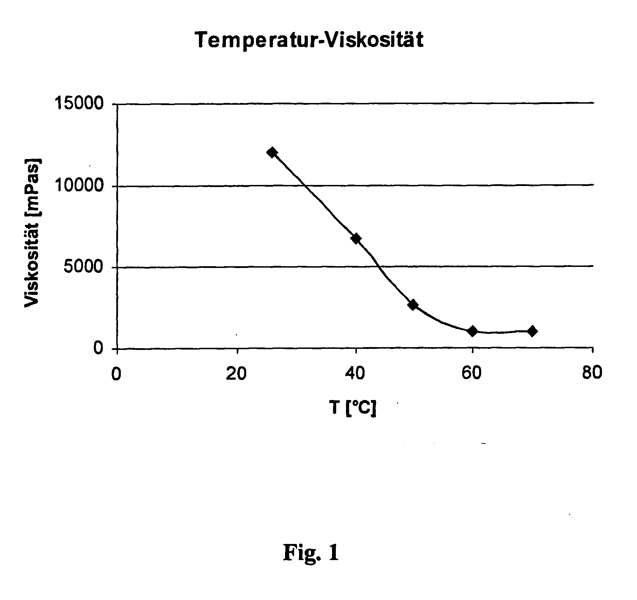

Das System aus Tab. 1 bildet, je nach Einstellung der Emulgatoren und/oder der Coemulgatoranteile, eine feste Struktur unterhalb einer Temperatur von 40°C, oberhalb dieser Temperatur (> 40 °C) liegt die Emulsion in flüssiger Form vor, da die Orientierung gestört wird (vgl. Fig. 1). Es handelt sich um eine sogenannte heiß-kalt-Emulgierung. Diese Bezeichnung beschreibt das Zusammenbringen einer heißen Ölphase (wegen der festen, aufzuschmelzenden Emulgatoranteile) und einer kalten, wässrigen Phase, wobei eine kinetisch stabile Öl-Wasser-Emulsion gebildet wird. Dies wird unterstützt durch die Zuführung mechanischer Energie (Scherenergie der Tröpfchen der inneren Phase - hier durch Homogenisieren).

**Tab. 1: Beispielformulierung Cremetuch**

| INCI | Funktion | Grund |
|---|---|---|
| Aqua | Basis | |
| Glycerin | Feuchtigkeit spendend | |
| Ceteth-20 | Emulgatoren | Bildung der flüssigkristalinen Phase |
| PEG-40 Stearate | | |
| Cetearyl Alcohol | | |
| Stearyl Alcohol | | |
| Glyceryl Stearate | | |
| Butyrospermum Parkii | Ölphase | Schmelztemperatur, Erstarrungstemperatur, Schmelzbereich |
| Caprylic/Capric Triglyceride | | |
| Isopropyl Palmitate | | |
| Dicaprylyl Carbonate | | |
| Cyclohexasiloxane | | |
| Cyclopentasiloxane | | |
| Parfum | Duft | |
| Polyaminopropyl Biguanide | Konservierungssystem | |
| Potassium Sorbate | | |
| Methylparaben | | |
| Propylparaben | | |
| Tocopheryl Actetate | Wirkstoffe | |
| Panthenol | | |
| Triisostearin | Mikroemulsion | Erhöhung der Stabilität in der flüssigen Phase |
| Octyldodecyl Lactate | | |
| Diglycerin | | |
| Oryza Sativa | | |
| Ceteareth-22 | | |
| C10-15 Alkyl Benzoate | | |
| Palmeth-2 | | |
| Cetyl Palmitate | | |
| Palmeth-2 Phosphate | | |
| Dimethicone | | |
| Phenoxyethanol | | |
| Benzoic Acid | | |
| Dehydroacetic Acid | | |
| Ethylhexylglycerin | | |

Die Beispielformulierung aus Tab. 1 wurde in den in Tab. 2 gezeigten Anteilen realisiert.

**Tab. 2: Ausgangsformulierung**

| **Bestandteil** | **Anteil [Gew.-%]** |
|---|---|
| Aqua | ad 100 |
| Glycerin | 2,65 |
| Ceteth-20 | 2,25 |
| Cetearyl Alcohol | 2,00 |
| Stearyl Alcohol | 2,00 |
| Glyceryl Stearate | 1,80 |
| Butyrospermum Parkii | 1,50 |
| Caprylic/Capric Triglyceride | 1,00 |
| Isopropyl Palmitate | 1,00 |
| Dicaprylyl Carbonate | 0,50 |
| Cyclohexasiloxane | 0,50 |
| Cyclopentasiloxane | 0,50 |
| PEG-40-Stearate | 0,30 |
| Polyaminopropyl Biguanide | 0,30 |
| Potassium Sorbate | 0,30 |
| Methylparaben | 0,25 |
| Tocopheryl Acetate | 0,20 |
| Propylparaben | 0,15 |
| Triisostearin | 0,09 |
| Octyldodecyl Lactate | 0,07 |
| Diglycerin | 0,05 |
| Oryza Sativa | 0,05 |
| Ceteareth-22 | 0,04 |
| C12-15 Alkyl Benzoate | 0,04 |
| Palmeth-2 | 0,03 |
| Cetyl Palmitate | 0,02 |
| Palmeth-2 Phosphate | 0,02 |
| Dimethicone | 0,02 |
| Phenoxyethanol | 0,01 |
| Benzoic Acid | 0,01 |
| Dehydroacetic Acid | 0,01 |
| Ethylhexylglycerin | 0,01 |

An Hand der Formulierung aus Tab. 2 wurden nun die charakteristischen Parameter genauer untersucht. Die Ergebnisse sind in Tab. 3 zusammengefasst.

**Tab. 3: Ergebnisse charakteristische Parameter Ausgangsformulierung**

| **Formulierung** | **Bezeichnung** | T_{S} [°C] | T_{E} [°C] | T_{Z} [°C] | dT_{S} [°C] |
|---|---|---|---|---|---|
| Tab. 2 | Ausgangsformulierung | 58 | 28 | 85 | 30 |

Der Wert T_{S} ist wichtig für die Verflüssigung der Creme vor Auftragung auf das Trägermaterial und die Temperatur T_{E} wiederum zur Beschreibung des Punktes, wann die Creme anfängt, ihre flüssigkristalline Phase zu bilden. Die Zersetzungstemperatur beschreibt den Punkt, an welchem die Creme irreversibel einer Phasentrennung unterliegen würde. Entscheidend für eine gute Verarbeitung ist der Bereich der Schmelztemperatur dT_{S}.

Mit diesen Erkenntnissen wurden die Bereiche Ölphase (Wachse) und die zugegebene Mikroemulsion zur Stabilisierung der Flüssigphase bei einer Temperatur oberhalb von T_{S} untersucht. Dazu wurde die Sheabutter (Butyrospermum Parkii) gegen unterschiedliche Jojobaester substituiert. Durch eine unterschiedliche Anzahl an Estereinheiten wurde der Schmelzpunkt der Ölkomponente variiert. Die Ergebnisse sind in Tab. 4 dargestellt. Der Parameter t_{E} (Erstarrungszeit) wurde hier zusätzlich beobachtet.

**Tab. 4: Ergebnisse Einfluss Ölkomponente**

| **Formulierung** | **Bezeichnung** | **T_{S} [°C]** | **T_{E} [°C]** | **t_{E} [min]** | **T_{Z} [°C]** | **dT_{S}** [°C] |
|---|---|---|---|---|---|---|
| Tab. 2 | Ausgangsform. | 58 | 28 | 35 | 85 | 30 |
| Jojobaester 10 | Ölphase 1 | 55 | 26 | 24 | 82 | 29 |
| Jojobaester 20 | Ölphase 2 | 60 | 28 | 29 | 75 | 32 |
| Jojobaester 30 | Ölphase 3 | 65 | 30 | 38 | 68 | 35 |

Es zeigte sich, dass ein direkt proportionaler Einfluss der Ölkomponente besteht: Je höher das Molekulargewicht der Ölkomponente ist, desto höher liegt auch die Schmelztemperatur und die Erstarrungstemperatur. Überraschender weise zeigte sich, dass der Schmelzbereich mit längerkettigen Ölanteilen (Wachsen) erhöht werden konnte (was in der Anwendung allerdings in Konkurrenz zur Zersetzungstemperatur T_{Z} steht). Die Erstarrungszeiten verhielten sich ebenfalls proportional zum Schmelzbereich.

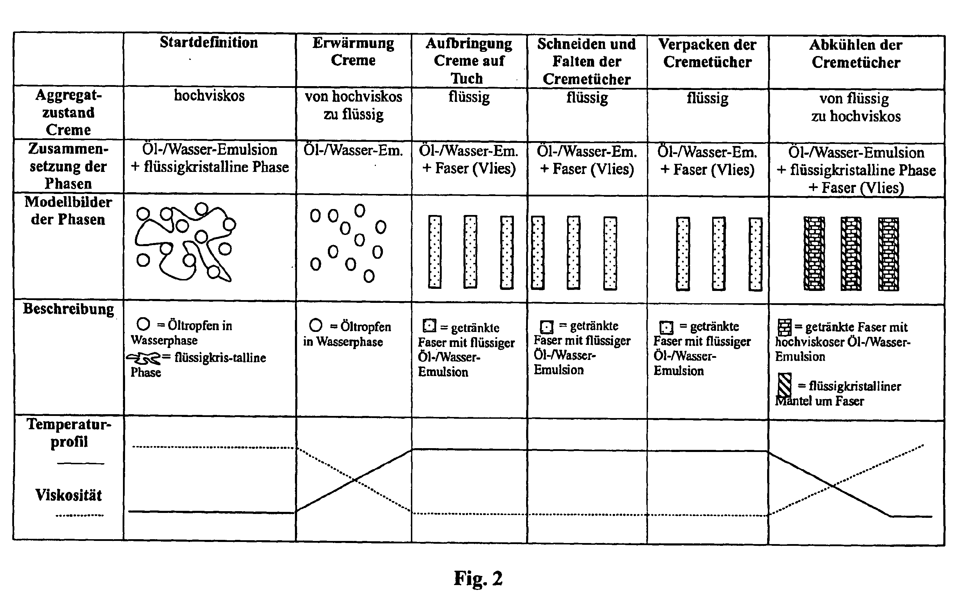

Mit der Ausgangsformulierung aus Tab. 2 können somit homogen getränkte Cremetücher hergestellt werden. Dazu wird die Creme auf ca. 60°C erwärmt, bis sie flüssig und die flüssigkristalline Phase aufgeschmolzen ist. Danach wird die verflüssigte Creme auf das Trägermaterial aufgebracht. Schliesslich wird das mit der Creme versehene Trägermaterial wieder abgekühlt, bis die Creme wieder hochviskos ist und wieder eine flüssigkristalline Phase umfaßt. Das Tränkungsmedium ist nun homogen über das Trägermaterial verteilt, indem das Tränkungsmedium vom Trägermaterial bei geringer Sättigung nur durch Adsorption in den Fasern, bei höherer Sättigung zusätzlich durch Bildung von flüssigkristallinen Phasen um die Fasern und bei vollständiger Sättigung zusätzlich durch Auffüllen der Zwischenräume im Trägermaterial aufgenommen wird. Dieser Prozess mit den entsprechenden Wirkungen ist in Fig. 2 dargestellt. Im folgenden sollen diese (und weitere) Effekte noch genauer erläutert werden.

Der Nachweis wurde durch Messungen/Aufnahmen mit Hilfe des Mikroskops erbracht. Bevor die Emulsion aus Tab. 2 auf dem Tuch (Cremetuch) untersucht wurde, sollte der Effekt des Viskositätsanstiegs durch weitere Messungen belegt werden. Bei Aufnahmen der Emulsion im Hellfeld waren die flüssigkristallinen Anteile bei einer Temperatur von 23 °C deutlich zu erkennen. Bei einer Temperatur von 60 °C waren diese Anteile in der Lotion nicht mehr zu beobachten.

Nun konnte der beschriebene Effekt in einem Feuchttuch (Cremetuch) beschrieben werden. Dabei zeigte sich, dass bei niedrigem Tränkungsgrad (Gewichtsverhältnis Lotion zu Trägermaterial von 2,7 : 1) die warme, flüssige Creme zunächst auch der Adsorption der Vlies- (o.ä.) Struktur unterliegt. Das konnte mikroskopischen Aufnahmen entnommen werden. Eine Ansammlung der Creme in den Zwischenräumen blieb hier jedoch aus. Dies bedeutet, dass durch das Aufbringen der Creme im erwärmten, flüssigen Zustand zunächst die Adsorption des Trägermaterials in seiner physikalischen Kraft (Kapillarkräfte) überwiegt.

In einem Feuchttuch, welches dem Stand der Technik entspricht, kann ein Trägermaterial jedoch nicht über das Maß der Adsorption hinaus befeuchtet/getränkt werden. Um so überraschender war hier das in verschiedenen Versuchsreihen herausgefundene Ergebnis, dass durch die Ausbildung der flüssigkristallinen Phase eine wesentlich höhere Benetzung des Tuchs erfolgen konnte.

Eine Untersuchung des noch mit der flüssigen Creme versehenen Tuches zeigte zunächst, dass mit steigender Tränkung nicht nur die Fasern, sondern auch die Leerräume ausgefüllt werden. Eine komplette Befeuchtung auch in den Leerräumen durch eine derart intensive Tränkung (Creme : Tuch = 7 : 1) wäre gemäß dem Stand der Technik nicht möglich. Abschließend wurde nun durch Kühlung des Cremetuchs auf 20 °C die Bildung der flüssigkristallinen Phase initiiert. Überraschenderweise stellte sich dabei heraus, dass sich die flüssigen Phasen nun auch in den Leerräumen der Strukturmatrix des Trägermaterials - d.h. auf den Fasern - ausgebildet hatten. Damit wurde der Beweis erbracht, dass sich die Viskosität der Creme nach erfolgter Abkühlung auch in einem Tuch reversibel steuern läßt.

Zwischen der kritischen Phasentemperatur T_{Z} und der Temperatur des Viskositätsanstiegs wurden diese Lotionen mit definierter Tränkungsmenge auf ein Tuch appliziert. Nach erfolgter Tränkung wurde das Tuch (oder der Tuchstapel) abgekühlt, und es bildete sich unter normalen Klimaverhältnissen die cremige Modifikation der Lotion.

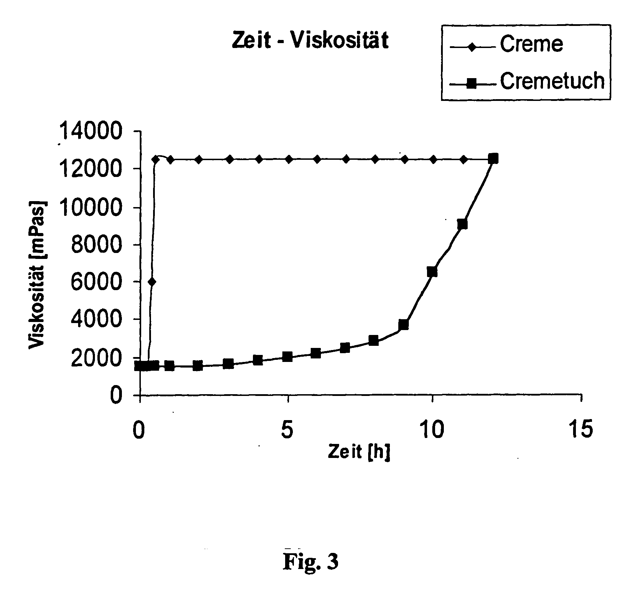

Die Verteilung ist bei dieser Herstellung nicht gleichwertig zu den bisher üblichen Systemen, da die Trägermaterialmatrix durch die flüssigkristalline Phase komplett aufgefüllt werden kann. Die Ausbildung der flüssigen Phase unterliegt, bedingt durch das Trägermaterial (Phase Trägermaterial), einer kinetischen Verzögerung. Dieses Verhalten entsprechend einer Hysterese zeigt sich deutlich in Fig. 3.

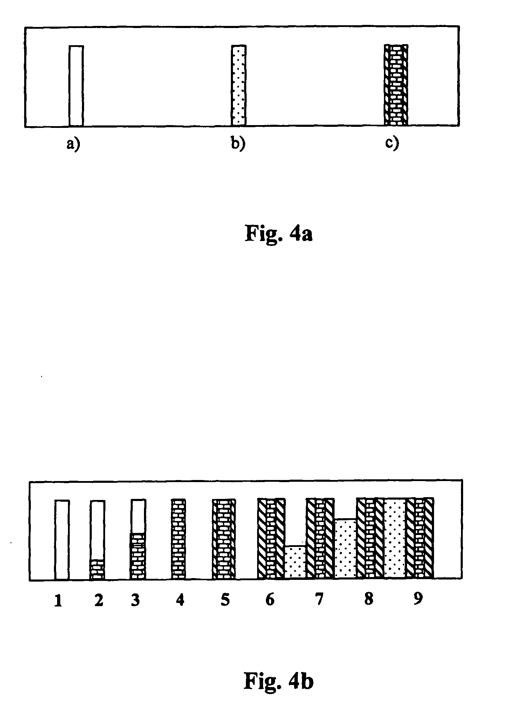

In Fig. 4a ist schematisch der Unterschied in den Tränkungsmöglichkeiten zwischen einem herkömmlichen Feuchttuch und dem erfindungsgemäßen Cremetuch dargestellt. Dabei stellt Säule a) die trockene Faser eines Vliesmaterials dar, Säule b) die mit einer üblichen Lotion (niedrigviskoses Medium) getränkte Faser eines Feuchttuchs (wobei die Aufnahme ausschließlich über die Adsorption des Trägermaterials erfolgt) und Säule c) den Faseraufbau eines Cremetuchs. Im Fall c) wird die Lotion nach dem Abkühlen zu einer Creme - wobei in Abhängigkeit vom Befeuchtungs- bzw. Sättigungsgrad flüssigkristalline Phasen auch außerhalb der Faser gebildet werden. Fig. 4b zeigt den oben bereits beschriebenen Effekt des Befeuchtungs- bzw. Sättigungsgrades in einem Cremetuch genauer. Säule 1 zeigt eine ungetränkte Faser und die Säulen 2-4 beschreiben die Adsorption des Tränkungsmediums. In diesem Zusammenhang sollte angemerkt werden, dass die Aufnahme der Creme entgegen der Darstellung des Modellbildes homogen erfolgt - durch die hier gezeigte Darstellung soll lediglich der Grad der Sättigung in Bezug auf die Adsorption dargestellt werden. Ab Säule 5 bilden sich Flüssigkristalle um die Faser. Die Säulen 6-9 geben die Sättigung des Tuchs außerhalb der Adsorption wieder (beschriebene Tränkung im Verhältnis Creme 7 zu 1 Gewichtsanteil Trägermaterial).

Abschließend wurde der Effekt der neuen Oberflächenmodifikation mittels Laserprofilometrie wiederholt nachgewiesen. Es wurden verschiedene feuchte Tücher (mit flüssiger Emulsion) und Cremetücher auf die Rauhigkeit der Oberfläche untersucht. Die auch in dieser Hinsicht überraschenden Ergebnisse sind in Tab. 5 zusammengefasst. Dabei ist R_{3z} = arithmetisches Mittel der 5 Einzelrauhtiefen (die Einzelrautiefe ist definiert als der senkrechte Abstand zwischen der dritthöchsten Profilspitze und dem dritttiefsten Profiltal innerhalb der Einzelmessstrecke des gefilterten Rauheitsprofils, wobei vertikale und horizonzale Mindestgrößen überschritten werden müssen); Rₘₐₓ = größte innerhalb der Gesamtmessstrecke vorkommende Einzelrautiefe des gefilterten Profils; und R_{z} = arithmetisches Mittel aus den Einzelrautiefen fünf aneinander grenzender, gleichlanger Einzelmessstrecken des gefilterten Profils.

**Tab. 5: Zusammenfassung der Rauhigkeit**

| **Feuchttuch** | **R_{3z} [µm]** | **Rₘₐₓ [µm]** | **R_{z} [µm]** |
|---|---|---|---|
| Spunlace 60gsm. trocken | 222,7 | 488,2 | 349,2 |
| Handelsübliches Feuchttuch A | 414,5 | 585,3 | 446,3 |
| Handelsübliches Feuchttuch B | 374,3 | 593,2 | 548,0 |
| Feuchttuch mit 6g Creme auf Spunlace 60gsm. | 367,9 | 638,6 | 508,6 |
| Feuchttuch mit 9g Creme auf Spunlace 60gsm. | 585,3 | 765,0 | 688,8 |

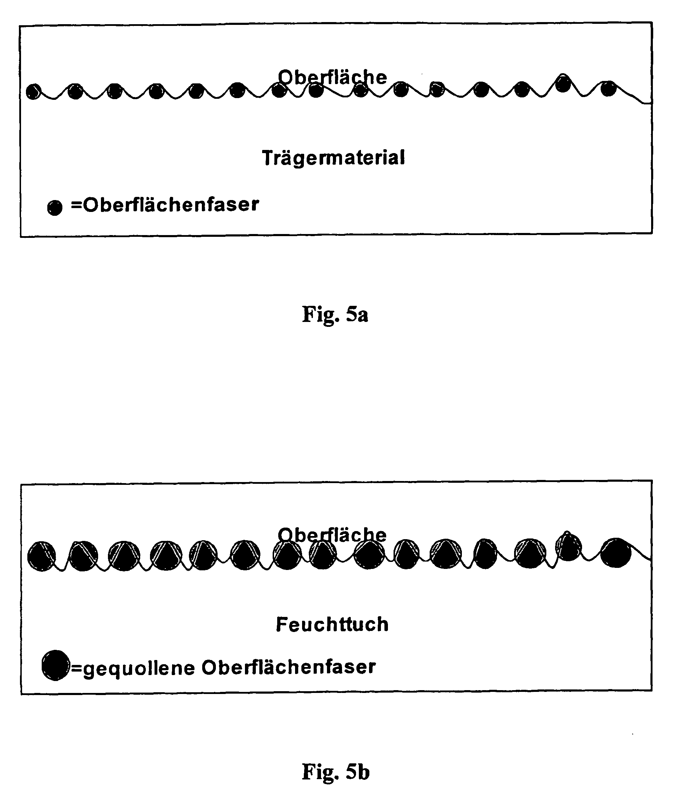

Es war zunächst nicht zu erwarten, dass die vertikale Rauhigkeit der Cremetücher höher ist als in einem üblichen Feuchttuch mit einer niedrigviskosen Lotion. Eine mögliche Erklärung ist die verspätete Bildung der flüssigkristallinen Phase, welche mit erhöhtem Anteil an Creme stärker ausgeprägt ist. Dies basiert wiederum auf der nachgewiesenen Adsorption im ersten Schritt. Erst mit dem Überschreiten der maximalen Adsorption findet eine Anlagerung der Creme mit steigendem Anteil außerhalb der Fasern statt. Die entsprechende Oberflächenstruktur ist jeweils in Fig. 5 a/b schematisch dargestellt. Dieser Effekt - steigende vertikale Rauhigkeit - wurde auch in Abhängigkeit von der Zeit beobachtet, da die Bildung der Flüssigkristalle einer zeitlichen Verzögerung unterliegen (vgl. Fig. 3). Bereits die handelsüblichen Feuchttücher nehmen durch die Tränkung in ihrer Rauhigkeit zu. Dies folgt aus der Adsorption der niedrigviskosen Lotion in den Fasern und dem damit verbundenen Quellungsprozess. In einem Cremetuch findet zusätzlich zur Adsorption in den Fasern die Ausbildung der Creme an der Grenzfläche der Fasern statt. Dadurch wird dieser Effekt entscheidend verstärkt.

Für die "sensitive" Wahrnehmung ist jedoch das Tränkungsmedium entscheidend. Das trockene Trägermaterial ist in der bestimmten vertikalen Rauhigkeit niedriger als die Feuchttücher, wird jedoch in jedem Anwendungstest als rauer empfunden. Daher ist das Tränkungsmedium für die Sensorik Ausschlag gebend. In den gefühlten Eigenschaften der Rauhigkeit erscheint das Cremetuch als wesentlich glatter. Dies ist zu erklären, wenn nicht nur die vertikale Rauhigkeit, sondern auch die Freiheitsgrade und deren Kraftvektoren dargestellt werden. Die Rauhigkeitsspitzen des Cremetuchs sind nicht sehr stabil. Dies bedeutet, dass die Rauhigkeit mit geringer Krafteinwirkung zerstört wird. Dieser minimale Kraftaufwand ist für den Menschen nicht wahrnehmbar. Vielmehr überwiegt die sensitive Wahrnehmung der Eigenschaften der Creme.

Neben den zusätzlich realisierten Eigenschaften des Pflegens durch Cremen ist es nunmehr auch möglich, das durch die Gravitation bedingte "Absacken" der Lotion zum Packungsboden hin zu unterbinden. Das Produkt erscheint nun homogener in seiner Produktverteilung und Zusammensetzung aus Träger und Creme.

Besonders geeignet sind die so entwickelten Cremetücher für die Intensivierung der Pflegeeigenschaften. Des weiteren ist es durch das erfindungsgemäße Cremetuch nun auch möglich, durch die Überwindung des physikalischen Parameters der Viskosität neue, weitere Rohstoffe und Wirkstoffe einzusetzen, welches bei den bisher üblichen niedrigviskosen Lotionen nicht möglich war. Die Eigenschaften der Hautverträglichkeit wurden ebenfalls verbessert, da nun durch die zusätzlichen Formulierungsmöglichkeiten reichhaltigerer Emulsionen auch ein temporär verstärkter Wirkungseffekt der Tücher gegeben ist.

Das Tränkungsmedium enthält vorteilhafterweise nicht-ionische Tenside oder kolloidale Bestandteile. Als besonders vorteilhaft haben sich beispielsweise folgende Substanzen erwiesen:
- Ceteth-20
- Ceteareth-20
- Ceteareth-22
- Polysorbate 60
- Steareth-100
- Steareth-20
- Steareth-10
- Steareth-2
- Sorbitan Stearate
- PEG-8 Distearate
- PEG-30 Stearate
- PEG-40 Stearate
- PEG-100 Stearate
mit
- Glyceryl Stearate
- Methylglucose Sesquistearate
- Cetearyl Alcohol
- Glyceryl Alcohol

Des weiteren haben sich im Bereich der Öle und Wachse beispielsweise folgende Komponenten als sehr vorteilhaft erwiesen:
- Mandelöl
- Jojobaester 10
- Jojobaester 20
- Jojobaester 30
- Sheabutter
- Olivenöl
- Jojobaöl
- Dicaprylyl Carbonate
- Caprylic/Capric Triglyceride

Es ist vorteilhaft, bei der vorliegenden Erfindung mit einem Ölanteil und Emulgatoranteil in Summe von 5 - 30% zu arbeiten. Der Anteil steht in Abhängigkeit vom resultierenden Flüssigkeitsfenster (Viskosität - Temperatur).

Die Zusammensetzungen enthalten gemäß der Erfindung außer den vorgenannten Substanzen gegebenenfalls die in der Kosmetik üblichen Zusatzstoffe. Diese sind beispielsweise Parfümöl, Konservierungsmittel, Pflanzenextrakte, Vitamine, Wirkstoffe, Antioxidantien, oder andere übliche Bestandteile einer kosmetischen oder dermatologischen Formulierung wie Alkohole, Glycerin, Polyether, Polymere oder Silikonderivate.

## Patentansprüche

1. Verfahren zur Herstellung eines Cremetuchs, das ein Trägermaterial und ein hochviskoses Tränkungsmedium mit einer Viskosität > 2000 mPas in Form einer Öl-/Wasser-Emulsion mit flüssigkristalliner Phase enthält, **gekennzeichnet durch** die folgenden Schritte:
- Erwärmen des hochviskosen Tränkungsmediums, bis es flüssig und die flüssigkristalline Phase aufgeschmolzen ist;
- Aufbringen des nunmehr flüssigen Tränkungsmediums auf das Trägermaterial;
- Abkühlen des mit dem Tränkungsmedium versehenen Trägermaterials, bis das Tränkungsmedium wieder hochviskos ist und wieder eine flüssigkristalline Phase umfaßt, sodaß das Tränkungsmedium homogen über das Trägermaterial verteilt ist, indem das Tränkungsmedium vom Trägermaterial bei geringer Sättigung nur **durch** Adsorption in den Fasern, bei höherer Sättigung zusätzlich **durch** Bildung von flüssigkristallinen Phasen um die Fasern und bei vollständiger Sättigung zusätzlich durch Auffüllen der Zwischenräume im Trägermaterial aufgenommen wird.

2. Verfahren nach Anspruch 1, **dadurch gekennzeichnet, dass** das Tränkungsmedium bei Temperaturen von < 40 °C in fester, cremiger oder pastenartiger Modifikation vorliegt.

3. Verfahren nach Anspruch 1 oder 2, **dadurch gekennzeichnet, dass** der Tränkungsanteil bei 100-700 Gew.-% Lotion/Tuch vorliegt.

4. Verfahren nach einem der vorangegangenen Ansprüche, **dadurch gekennzeichnet, dass** das Trägermaterial ein Gewicht von 30-120 g/m² hat.

5. Cremetuch, umfassend ein Trägermaterial und ein hochviskoses Tränkungsmedium mit einer Viskosität > 2000 mPas in Form einer Öl-/Wasser-Emulsion mit flüssigkristalliner Phase, **dadurch gekennzeichnet, dass** das Tränkungsmedium homogen über das Trägermaterial verteilt ist, indem das Tränkungsmedium vom Trägermaterial bei geringer Sättigung nur durch Adsorption in den Fasern, bei höherer Sättigung zusätzlich durch Bildung von flüssigkristallinen Phasen µm die Fasern und bei vollständiger Sättigung zusätzlich durch Auffüllen der Zwischenräume im Trägermaterial verteilt ist.

6. Cremetuch nach Anspruch 5, **dadurch gekennzeichnet, dass** das Tränkungsmedium bei Temperaturen von < 40 °C in fester, cremiger oder pastenartiger Modifikation vorliegt.

7. Cremetuch nach Anspruch 5 oder 6, **dadurch gekennzeichnet, dass** der Tränkungsanteil bei 100-700 Gew.-% Lotion/Tuch vorliegt.

8. Cremetuch nach einem der Ansprüche 5-7, **dadurch gekennzeichnet, dass** das Trägermaterial ein Gewicht von 30-120 g/m² hat.

## Claims

1. A method of making a cream cloth comprising a carrier material and a high-viscosity impregnation medium of higher than 2000 mPas viscosity in the form of an oil-in-water emulsion having a liquid crystalline phase, **characterized by** the following steps:
- Heating the high-viscosity impregnation medium until liquid and the liquid crystalline phase has melted;
- applying the now liquid impregnation medium to the carrier material;
- cooling the carrier material with the impregnation medium applied thereto until the latter is highly viscous once more and again includes a liquid crystalline phase so that the impregnation medium is homogenously distributed through the carrier material in such a way that the impregnation medium is received by the carrier material by adsorption in the fibers if the saturation is low, by the additional formation of liquid crystalline phases surrounding the fibers if the saturation is higher, and by additionally filling the interstices in the carrier material if the saturation is total.

2. Method as in claim 1, **characterized in that**, at temperatures lower than 40 °C, the impregnation medium is present in a solid, creamy or paste-like modification.

3. Method as in claim 1 or 2, **characterized by** the impregnant proportion being approximately 100 wt.% to 700 wt.% of lotion per cloth.

4. Method as in any one of the preceding claims, **characterized by** the carrier material having a weight of 30 g/m² to 120 g/m².

5. Cream cloth, comprising a carrier material and a high-viscosity impregnation medium of higher than 2000 mPas viscosity in the form of an oil-in-water emulsion having a liquid crystalline phase, **characterized by** the impregnation medium being distributed homogenously through the carrier material by being distributed through the carrier material solely by the adsorption in the fibers if the saturation is low, by the additional formation of liquid crystalline phases surrounding the fibers if the saturation is higher, and by additionally filling the interstices in the carrier material if the saturation is total.

6. Cream cloth as in claim 5, **characterized in that**, at temperatures lower than 40 °C, the impregnation medium is present in a solid, creamy or paste-like modification.

7. Cream cloth as in claim 5 or 6, **characterized by** the impregnant proportion being approximately 100 wt.% to 700 wt.% of lotion per cloth.

8. Cream cloth as in any one of claims 5 to 7, **characterized by** the carrier material having a weight of 30 g/m² to 120 g/m².

## Revendications

1. Procédé de fabrication d'une lingette imprégnée de crème, qui renferme un support et un agent d'imprégnation très visqueux, d'une viscosité > 2000 mPas, en forme d'une émulsion huile/eau avec une phase cristalline liquide, **caractérisé par** les étapes suivantes :
- chauffage de l'agent d'imprégnation très visqueux, jusqu'à ce qu'il soit liquide et que la phase cristalline liquide soit fondue ;
- application de l'agent d'imprégnation désormais liquide sur le support ;
- refroidissement du support muni de l'agent d'imprégnation, jusqu'à ce que l'agent d'imprégnation soit de nouveau très visqueux et comporte une phase cristalline liquide, de sorte que l'agent d'imprégnation est réparti de façon homogène sur le support, l'agent d'imprégnation n'étant absorbé par le support, pour une saturation relativement faible, que par adsorption dans les fibres, en supplément, pour une saturation plus élevée, par formation de phases cristallines liquides autour des fibres, et en supplément, pour une saturation complète, par remplissage des interstices dans le support.

2. Procédé suivant la revendication 1, **caractérisé en ce que** l'agent d'imprégnation est présent en modification solide, crémeuse ou pâteuse, à des températures < 40°C.

3. Procédé suivant l'une des revendications 1 et 2, **caractérisé en ce que** la part d'imprégnation est présente pour 100 - 700 % en poids de lotion/lingette.

4. Procédé suivant l'une des revendications précédentes, **caractérisé en ce que** le support a un poids de 30 - 120 g/m².

5. Lingette imprégnée de crème, comprenant un support et un agent d'imprégnation très visqueux, d'une viscosité > 2000 mPas, en forme d'une émulsion huile/eau avec une phase cristalline liquide, **caractérisée en ce que** l'agent d'imprégnation est réparti de façon homogène sur le support, l'agent d'imprégnation n'étant réparti par le support, pour une saturation relativement faible, que par adsorption dans les fibres, en supplément, pour une saturation plus élevée, par formation de phases cristallines liquides autour des fibres, et en supplément, pour une saturation complète, par remplissage des interstices dans le support.

6. Lingette imprégnée dé crème suivant la revendication 5, **caractérisée en ce que** l'agent d'imprégnation est présent en modification solide, crémeuse ou pâteuse, à des températures < 40°C.

7. Lingette imprégnée de crème suivant l'une des revendications 5 et 6, **caractérisée en ce que** la part d'imprégnation est présente pour 100 - 700% en poids de lotion/lingette.

8. Lingette imprégnée de crème suivant l'une des revendications 5 à 7, **caractérisée en ce que** le support a un poids de 30 à 120 g/m².
